(19) Europäisches Patentamt
European Patent Office
Office européen des brevets

(11) Publication number: **0 052 339**
**A1**

(12) **EUROPEAN PATENT APPLICATION**

(21) Application number: 81109625.4

(22) Date of filing: 11.11.81

(51) Int. Cl.³: C 07 D 239/62

(30) Priority: 13.11.80 JP 160183/80

(43) Date of publication of application: 26.05.82 Bulletin 82/21

(84) Designated Contracting States: CH DE FR LI

(71) Applicant: Daikin Kogyo Co., Ltd.
No 1-12-39, Umeda Kita-ku
Osaka-shi Osaka-fu(JP)

(72) Inventor: Ishikawa, Nobuo
10-10- Shinoharadai-machi
Kohoku-ku Yokohama, Kanagawa(JP)

(74) Representative: von Kreisler, Alek, Dipl.-Chem. et al,
Deichmannhaus am Hauptbahnhof
D-5000 Köln 1(DE)

(54) **Fluorobarbituric acid derivatives and their preparation.**

(57) A 5-fluorobarbituric acid derivative of the formula:

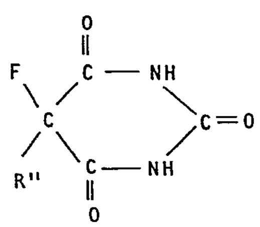

wherein R″ is a hydrogen atom or a $C_1$-$C_{10}$ hydrocarbon group has a physiological activity and can be prepared by reacting dialkyl 2-fluoro-2-substituted or unsubstituted-malonate of the formula:

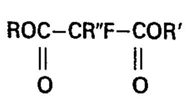

wherein R and R′ are each a lower alkyl group and R″ is as defined above, with urea in the presence of an alkali metal alkoxide.

EP 0 052 339 A1

# FLUOROBARBITURIC ACID DERIVATIVES AND THEIR PREPARATION

This invention relates to fluorobarbituric acid derivatives and their preparation.

The said fluorobarbituric acid derivatives are representable by the formula:

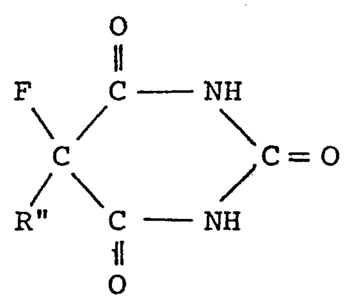

(I)

wherein R" is a hydrogen atom or a $C_1$-$C_{10}$ hydrocarbon group such as alkyl (preferably $C_1$ - $C_5$ alkyl), alkenyl (preferably $C_2$ - $C_5$ alkenyl), cycloalkyl (preferably $C_3$ - $C_7$ cycloalkyl), cycloalkenyl (preferably $C_3$ - $C_7$ cycloalkenyl) or aralkyl (preferably phenyl ($C_1$ - $C_3$) alkyl).

Specific examples of the fluorobarbituric acid derivatives (I) are 5-fluorobarbituric acid, 5-fluoro-5-alkyl-barbituric acid (e.g. 5-fluoro-5-methyl-barbituric acid, 5-fluoro-5-ethylbarbituric acid, 5-fluoro-5-butylbarbituric acid), 5-fluoro-5-cycloalkylbarbituric acid (e.g. 5-fluoro-5-cyclopentylbarbituric acid, 5-fluoro-5-cyclohexylbarbituric acid), 5-fluoro-5-alkenylbarbituric acid (e.g. 5-fluoro-5-allylbarbituric acid), 5-fluoro-5-aralkylbarbituric acid (e.g. 5-fluoro-5-benzylbarbituric acid, 5-fluoro-5-phenethylbarbituric acid), etc.

According to this invention, the fluorobarbituric

acid derivative (I) may be prepared by reacting dialkyl 2-fluoro-2-substituted or unsubstituted-malonate of the formula:

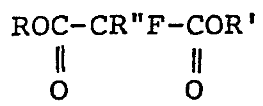
(II)

wherein R and R' are each a lower alkyl group (preferably $C_1 - C_5$ alkyl) and R" is as defined above, with urea in the presence of an alkali metal alkoxide.

Dialkyl 2-fluoro-2-substituted or unsubstituted-malonate (II) to be used as the starting material may be prepared, for example, by the following process:

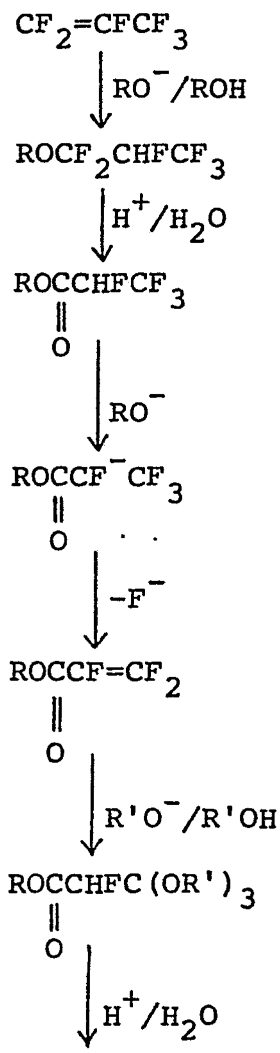

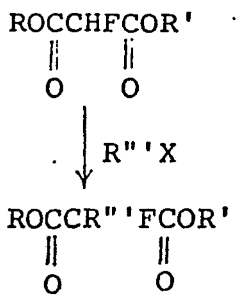


(IIa)

(IIb)

wherein X is a halogen atom, R, R' and R" are each as defined above and R"' is a $C_1$ - $C_{10}$ hydrocarbon group.

The cyclization reaction in the process of the invention can proceed by treatment of urea with dialkyl 2-fluoro-2-substituted or unsubstituted-malonate (II) in the presence of an alkoxide ion at a temperature from room temperature to elevated temperature in a solvent. The molar ratio of the urea and the dialkyl 2-fluoro-2-substituted or unsubstituted-malonate (II) may be usually from 1 : 1 to 2 : 1. As the source of the alkoxide ion, an alkali metal alkoxide is usually employed, and its specific examples are sodium methoxide, potassium methoxide, sodium ethoxide, potassium ethoxide, sodium propoxide, potassium propoxide, sodium butoxide, etc. As the solvent, an alcohol, preferably a lower alkanol such as methanol or ethanol, may be used. The reaction temperature is usually from room temperature to the boiling point of the solvent.

The desired product may be isolated from the reaction mixture and purified by per se conventional separation and purification procedures.

The fluorobarbituric acid derivatives (I) show a physiological activity such as hypnotic activity and are

useful as hypnotic agents. They are also useful as intermediates in the production of other per se physiologically active substances.

The present invention will be hereinafter explained further in detail by the following Examples.

Example 1

5-Fluorobarbituric acid:-

A mixture of dimethyl 2-fluoromalonate (1.4 g), urea (1.2 g) and sodium methoxide (0.54 g) in methanol (5 ml) was refluxed for five hours. Methanol was then evaporated off. The residue was recrystallized from aqueous ethanol to give 5-fluorobarbituric acid (1.1 g). Yield: 78%. M.P. > 250°C. $^{19}F$ NMR spectrum δ (ppm): 127.5 (using trifluoroacetic acid as the external standard). Mass spectrum (m/e): 146 (M+).

Exapmles 2 to 6

In the same manner as in Example 1 but using dimethyl 2-fluoro-2-R"-malonate wherein R" is as shown in Table 1 in place of 2-fluorobarbituric acid, the corresponding 5-fluoro-5-R"-barbituric acid was prepared. Yields were as shown in Table 1.

Table 1

| Example No. | R" | Yield (%) | Melting point (°C) | $^{19}F$ NMR δ (ppm) *1) | Mass spectrum (m/e) |
|---|---|---|---|---|---|
| 2 | $CH_3$- | 44 | >250 | 65.5 | 150 (M+) |
| 3 | $C_2H_5$- | 40 | >250 | 77.0 | 164 (M+) |
| 4 | $C_4H_9$- | 72 | >250 | 76.5 | 202 (M+) |
| 5 | $CH_2$=$CHCH_2$- | 65 | >250 | 74.5 | 186 (M+) |
| 6 | 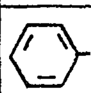-$CH_2$- | 55 | >250 | 74.0 | 236 (M+) |

Note: *1) Using trifluoroacetic acid as the external standard.

What is claimed is:

1. A compound of the formula:

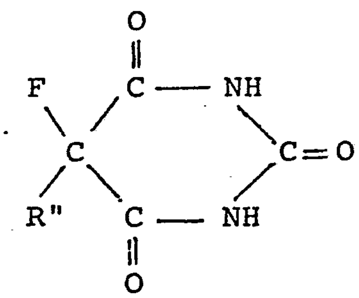

wherein R" is a hydrogen atom or a $C_1$ - $C_{10}$ hydrocarbon group.

2. The compound according to claim 1 wherein R" is a hydrogen atom.

3. The compound according to claim 1 wherein R" is alkyl, alkenyl, cycloalkyl, cycloalkenyl or aralkyl, the carbon atoms in those groups being not more than 10.

4. The compound according to claim 1 wherein R" is methyl, ethyl or butyl.

5. The compound according to claim 1 wherein R" is cyclopentyl or cyclohexyl.

6. The compound according to claim 3 wherein R" is benzyl or phenethyl.

7. A process for preparing a compound of the formula:

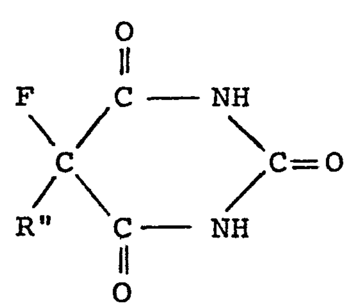

wherein R" is a hydrogen atom or a $C_1$ - $C_{10}$ hydrocarbon group, which comprises reacting dialkyl 2-fluoro-2-substituted or unsubstituted-malonate of the formula:

```
ROC-CR"F-COR'
 ‖       ‖
 O       O
```

wherein R and R' are each a lower alkyl group and R" is as defined above, with urea in the presence of an alkali metal alkoxide.

8. The process according to claim 7 wherein the reaction is effected in the presence of a solvent.

9. The process according to claim 8 wherein the solvent is a lower alkanol.

10. The process according to claim 7 wherein the reaction is effected at a temperature of from room temperature to the boiling point of the solvent.

11. The process according to claim 7 wherein the alkali metal alkoxide is one selected from the group consisting of sodium methoxide, potassium methoxide, sodium ethoxide, potassium ethoxide, sodium propoxide, potassium propoxide and sodium butoxide.

0052339

European Patent Office

**EUROPEAN SEARCH REPORT**

Application number

EP 81 10 9625

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, volume 81, 1974, page 389, abstract 49644z COLUMBUS OHIO (US) & Khim. Geterotsikl. Soedin. 1974, (4), 552-3 I.V. VIGALOK et al. "5-fluoro-5-alkyl(aryl)barbituric acids"<br>* abstract * | 1-6 | C 07 D 239/62 |
| X | TETRAHEDRON LETTERS, volume 21. (1980) OXFORD (GB) S.S. YEMUL et al. "Selective fluorination by $C_{19}XeF_6$" pages 277-280<br>* page 277, 278 above 279, table 1 * | 1,2 | TECHNICAL FIELDS SEARCHED (Int.Cl. 3)<br>C 07 D 239/00 |
| X | US - A - 3 030 408 (Ch. INMAN et al.)<br>* column 1, column 6, examples 5,6 * | 1-4, 7-11 | |

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document

X The present search report has been drawn up for all claims

| Place of search | Date of completion of the search | Examiner |
|---|---|---|
| The Hague | 19-02-1982 | FRANCOIS |

EPO Form 1503.1 06.78